(19) Europäisches Patentamt
European Patent Office
Office européen des brevets

(11) EP 4 124 337 A1

(12) EUROPEAN PATENT APPLICATION
published in accordance with Art. 153(4) EPC

(43) Date of publication:
**01.02.2023 Bulletin 2023/05**

(21) Application number: **20923195.0**

(22) Date of filing: **13.07.2020**

(51) International Patent Classification (IPC):
*A61K 31/4748* (2006.01) *A61P 31/14* (2006.01)
*A61P 9/12* (2006.01) *A61K 9/20* (2006.01)
*A61K 9/08* (2006.01) *A61K 9/72* (2006.01)
*C07D 491/18* (2006.01) *C07D 491/153* (2006.01)

(86) International application number:
**PCT/CN2020/101581**

(87) International publication number:
**WO 2021/174741 (10.09.2021 Gazette 2021/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.03.2020 CN 202010140356**

(71) Applicants:
- **Academy of Military Medical Sciences
Beijing 100850 (CN)**
- **Zhejiang Jinhua Conba Bio-Pharm. Co., Ltd.
Jinhua, Zhejiang 321016 (CN)**

(72) Inventors:
- **ZHONG, Wu
Beijing 100850 (CN)**
- **QIN, Chengfeng
Beijing 100850 (CN)**
- **ZHOU, Xinbo
Beijing 100850 (CN)**
- **DENG, Yongqiang
Beijing 100850 (CN)**
- **HU, Jiqiang
Beijing 100850 (CN)**
- **JIN, Qingping
Beijing 100850 (CN)**
- **YU, Bin
Beijing 100850 (CN)**
- **CAO, Ruiyuan
Beijing 100850 (CN)**
- **WANG, Manli
Beijing 100850 (CN)**
- **LI, Xiaofeng
Beijing 100850 (CN)**
- **ZHANG, Nana
Beijing 100850 (CN)**
- **FAN, Shiyong
Beijing 100850 (CN)**
- **HU, Zhihong
Beijing 100850 (CN)**
- **LI, Song
Beijing 100850 (CN)**

(74) Representative: **Böhm, Brigitte
Weickmann & Weickmann
Patent- und Rechtsanwälte PartmbB
Postfach 860 820
81635 München (DE)**

Remarks:
The complete document including Reference Table(s) and the Sequence Listing(s) can be downloaded from the EPO website

# (54) APPLICATION OF DIMETHYL BERBAMINE COMPOUND IN INHIBITION OF SARS-COV-2

(57) A compound represented by the general Formula I, a geometric isomer thereof, or a pharmaceutically acceptable salt thereof, and/or a solvate thereof, and/or a hydrate thereof for preventing and/or treating a pulmonary disease or symptom associated with SARS-CoV-2 or an asymptomatic or symptomatic SARS-CoV-2 infection, and an application of the compound represented by the general Formula I, the geometric isomer thereof, or the pharmaceutically acceptable salt thereof, and/or the solvate thereof, and/or the hydrate thereof in preparation of a product for preventing and/or treating a pulmonary disease or symptom associated with SARS-CoV-2 or asymptomatic or symptomatic SARS-CoV-2 infection,

EP 4 124 337 A1

## Description

The present invention is based on and claims the benefit of priority from Chinese application No. 202010140356.0, filed on March 3, 2020, the disclosure of which is incorporated herein by reference in its entirety.

## Technical Field

The present application belongs to the field of chemical drugs, and specifically relates to a use of a dimethylberbamine compound represented by Formula I, a geometric isomer thereof or a pharmaceutically acceptable salt and/or solvate and/or hydrate thereof, and a pharmaceutical composition comprising the above-mentioned compound in the treatment of a SARS-CoV-2 infection.

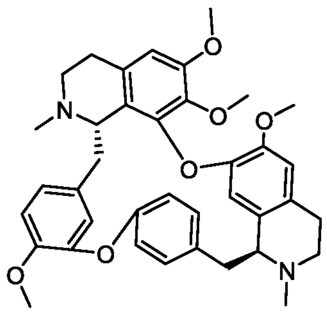

I

## Background Art

The dimethylberbamine compound represented by Formula I, with the chemical name of 6,6',7,12-tetramethoxy-2,2'-dimethylberbamine, is a bisbenzylisoquinoline alkaloid extracted from Stephania tetrandra. The compound of Formula I is also called Tetrandrine, and used as an antihypertensive drug, it is mainly used in the treatment of mild and moderate hypertension, can also be used in hypertensive crisis. In addition, it can also be used for rheumatism, joint pain, neuralgia. It is used for lung cancer in combination with low-dose of radiation; also used for simple silicosis of stage I, II, or III and coal silicosis of various stages.

Coronavirus is an enveloped, non-segmented, single-stranded, positive sense RNA virus with a wide range of animal hosts. SARS-CoV and MERS-CoV derived from zoonotic diseases can cause death in humans and broke out in 2002 and 2012, respectively (Zaki et al., 2012; Zhong et al., 2003; Cui et al., 2019). The 2019 novel coronavirus (2019-nCoV) is a new coronavirus strain that has never been found in humans before. On February 11, 2020, the International Committee on Taxonomy Viruses (ICTV) announced that the official name of 2019 novel Coronavirus (2019-nCoV) is called severe acute respiratory syndrome coronavirus 2 (SARS-CoV-2). On the same day, the World Health Organization (WHO) announced that the official name of the disease caused by this virus is COVID-19. The clinical manifestations of COVID-19 patients are that more than 90% of patients have fever, 80% of patients have dry cough, 20% of patients have shortness of breath, 15% of patients have dyspnea, and the most important manifestation is the reduction of white blood cells and lymphocytes, the patients' lungs have different degrees of organ damage, and there is a potential risk of developing pulmonary fibrosis. The virus is highly contagious, and SARS-CoV-2 infection events have occurred in many countries around the world, including Japan, South Korea, Italy, and the United States, and the trend continues to expand. Previous studies have found that drugs such as remdesivir and chloroquine phosphate can effectively inhibit the replication of SARS-CoV-2 virus (Wang et al., 2020), and relevant clinical studies have also been carried out. The related vaccines are also being developed. However, no drug or vaccine has been currently approved for widespread use.

Therefore, it is still very urgent and necessary to develop products that can effectively inhibit SARS-CoV-2.

## Contents of the present invention

The present inventors found in the research that tetrandrine can effectively inhibit SARS-CoV-2 virus, prevent or treat pulmonary fibrosis, thereby providing a new way and option for effective prevention and/or treatment of a pulmonary disease or symptom caused by SARS-CoV-2 virus. This application is now completed based on the above research.

The present application relates to a compound represented by Formula I, a geometric isomer thereof or a pharmaceutically acceptable salt and/or solvate and/or hydrate thereof, or a pharmaceutical composition comprising a

compound represented by Formula I, a geometric isomer thereof or a pharmaceutically acceptable salt and/or solvate and/or hydrate thereof, for use in the prevention and/or treatment of a pulmonary disease or symptom associated with SARS-CoV-2 or an asymptomatic or symptomatic SARS-CoV-2 infection,

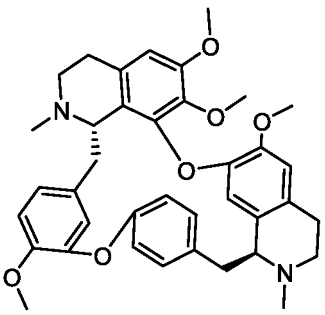

I.

The present application further relates to a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof, for use in the prevention and/or treatment of a pulmonary disease or symptom associated with SARS-CoV-2 and accompanied with hypertension, or an asymptomatic or symptomatic SARS-CoV-2 infection accompanied with hypertension,

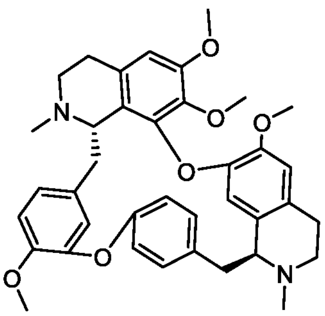

I.

The present application further relates to a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof, for use in the prevention and/or treatment of a COVID-19 or a COVID-19 accompanied with hypertension, or for use as a SARS-CoV-2 inhibitor, or for use in inhibiting the replication or reproduction of SARS-CoV-2 in a cell (e.g., a cell of mammal),

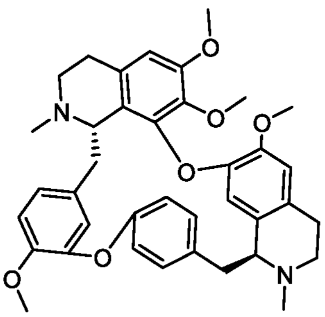

I.

The present application further relates to a compound represented by Formula I, a geometric isomer thereof

or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof, for use in the prevention and/or treatment of a disease or infection caused by a coronavirus.

The present application further relates to a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof, for use in the prevention and/or treatment of a disease or infection caused by a coronavirus and accompanied with hypertension.

The present application further relates to a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof, for use in the prevention and/or treatment of a disease or infection caused by a coronavirus or a disease or infection caused by a coronavirus and accompanied with hypertension.

The present application further relates to a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof, for use in the prevention and/or treatment of a pulmonary disease or symptom associated with coronavirus and accompanied with hypertension.

The present application further relates to a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof, for use in inhibiting the replication or reproduction of a coronavirus in a cell (e.g., a cell of mammal).

The present application further relates to a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof, for use as a coronavirus inhibitor.

The present application further relates to a use of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof in the manufacture of a product for the prevention and/or treatment of a disease or infection caused by a coronavirus.

The present application further relates to a use of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof in the manufacture of a product for the prevention and/or treatment of a disease or infection caused by a coronavirus and accompanied with hypertension.

The present application further relates to a use of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof in the manufacture of a product for the prevention and/or treatment of a pulmonary disease or symptom associated with a coronavirus and accompanied with hypertension.

The present application further relates to a use of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof in the manufacture of a product for inhibiting the replication or reproduction of a coronavirus in a cell (e.g., a cell of mammal).

The present application further relates to a use of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof in the manufacture of a product as a coronavirus inhibitor.

The present application further relates to a use of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof in the manufacture of a product for the prevention and/or treatment of a pulmonary disease or symptom associated with SARS-CoV-2, or an asymptomatic or symptomatic SARS-CoV-2 infection.

The present application further relates to a use of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt

and/or solvate and/or hydrate thereof in the manufacture of a product for the prevention and/or treatment of a pulmonary disease or symptom associated with SARS-CoV-2 and accompanied with hypertension, or an asymptomatic or symptomatic SARS-CoV-2 infection accompanied with hypertension.

The present application further relates to a use of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof in the manufacture of a product for the prevention and/or treatment of a COVID-19 or a COVID-19 accompanied with hypertension, or in the manufacture of a product as a SARS-CoV-2 inhibitor, or in the manufacture of a product for inhibiting the replication or reproduction of SARS-CoV-2 in a cell (e.g., a cell of mammal).

The present application further relates to a method for preventing and/or treating a disease or infection caused by a coronavirus or a disease or infection caused by a coronavirus and accompanied with hypertension, comprising administering to a host in need thereof a prophylactically and/or therapeutically effective amount of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof.

The present application further relates to a method for preventing and/or treating a pulmonary disease or symptom associated with a coronavirus or a pulmonary disease or symptom associated with a coronavirus and accompanied with hypertension, comprising administering to a host in need thereof a prophylactically and/or therapeutically effective amount of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof.

The present application further relates to a method for inhibiting the replication or reproduction of a coronavirus in a host in need thereof, comprising administering to the host in need thereof a prophylactically and/or therapeutically effective amount of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof.

The present application also relates to a method for preventing and/or treating a pulmonary disease or symptom associated with SARS-CoV-2 or an asymptomatic or symptomatic SARS-CoV-2 infection, comprising administering a prophylactically and/or therapeutically effective amount of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof to a host with the pulmonary disease or symptom associated with SARS-CoV-2 or a host with the asymptomatic or symptomatic SARS-CoV-2 infection.

The present application also relates to a method for preventing and/or treating a pulmonary disease or symptom associated with SARS-CoV-2 and accompanied with hypertension or an asymptomatic or symptomatic SARS-CoV-2 infection accompanied with hypertension, comprising administering a prophylactically and/or therapeutically effective amount of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof to a host with the pulmonary disease or symptom associated with SARS-CoV-2 and with hypertension or a host with the asymptomatic or symptomatic SARS-CoV-2 infection and with hypertension.

The present application also relates to a method for treating and/or preventing COVID-19 or COVID-19 accompanied with hypertension in a host in need thereof, or a method for inhibiting the replication or reproduction of SARS-CoV-2 in a host in need thereof, the method comprising administering to the host in need thereof a prophylactically and/or therapeutically effective amount of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof.

In some embodiments, the coronavirus described herein is SARS-CoV-2.

In some embodiments, the coronavirus described herein is SARS-CoV-2.

In some embodiments, the disease caused by the coronavirus described herein is COVID-19.

In some embodiments, the disease caused by the coronavirus and accompanied with hypertension described herein is COVID-19 accompanied with hypertension.

In some embodiments, the pulmonary disease or symptom associated with the coronavirus described herein is a pulmonary disease or symptom associated with SARS-CoV-2.

In some embodiments, the pulmonary disease or symptom associated with the coronavirus and accompanied with hypertension described herein is a pulmonary disease or symptom associated with SARS-CoV-2 and accompanied with hypertension.

In some embodiments, the infection caused by the coronavirus described herein is an asymptomatic or symptomatic SARS-CoV-2 infection.

In some embodiments, the infection caused by the coronavirus and accompanied with hypertension described herein is an asymptomatic or symptomatic SARS-CoV-2 infection accompanied with hypertension.

In some embodiments, the product described herein is a human drug or a veterinary drug for animal.

In some embodiments, the product described herein is a solid preparation, injection, spray, liquid preparation, inhalation preparation or compound preparation.

In some embodiments, the host described herein is a mammal.

In some embodiments, the mammal described herein includes bovine, equine, caprinae, suidae, canine, feline, rodent, primate, in which the preferred mammal is human, dog or pig.

In some embodiments, the COVID-19 described herein is a disease caused by SARS-CoV-2.

In some embodiments, the pharmaceutical composition described herein further comprises a pharmaceutically acceptable carrier or excipient. Specifically, the pharmaceutical composition can be a solid preparation, injection, inhalation preparation, spray, liquid preparation, or compound preparation.

The pharmaceutical composition described in the present application can be prepared into various forms according to different administration routes, such as oral tablet, capsule, powder, oral liquid, injection and transdermal preparation. According to conventional pharmaceutical practice, the pharmaceutically acceptable carrier includes diluent, filler, disintegrating agent, wetting agent, lubricant, coloring agent, flavoring agent or other conventional additive. Typical pharmaceutically acceptable carriers include, for example, microcrystalline cellulose, starch, crospovidone, povidone, polyvinylpyrrolidone, maltitol, citric acid, sodium lauryl sulfonate or magnesium stearate, and the like.

According to the application, the pharmaceutical composition can be administered in any of the following ways: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration via an explanted reservoir. Of these, the oral, intraperitoneal or intravenous administration is preferred.

For oral administration, the compound represented by Formula I, a geometric isomer thereof, or a pharmaceutically acceptable salt and/or solvate and/or hydrate thereof can be made into any orally acceptable preparation form, including but not limited to tablet, capsule, aqueous solution or suspension. Among them, the commonly used carrier for tablet includes lactose and corn starch, and lubricant such as magnesium stearate may also be added. Commonly used diluent for capsule preparation includes lactose and dried cornstarch. Aqueous suspension is usually prepared by mixing an active ingredient with suitable emulsifying agent and suspending agent. If necessary, some sweetening, flavoring agent or coloring agents may also be added to the above oral preparation form.

For rectal administration, the compound represented by Formula I, a geometric isomer thereof, or a pharmaceutically acceptable salt and/or solvate and/or hydrate thereof can generally be made into a suppository form, which is generally obtained by mixing the drug with a suitable non-irritating excipient. The excipient is solid at room temperature, but melts at rectal temperature to release the drug. Such excipient includes cocoa butter, beeswax and polyethylene glycol.

For topical administration, especially when treating an affected surface or organ that is easily accessible by topical application, such as eye, skin or lower intestinal neurological disease, the compound represented by Formula I, a geometric isomer thereof, or a pharmaceutically acceptable salt and/or solvate and/or hydrate thereof can be made into different topical preparation forms according to different affected surfaces or organs, and the specific description is as follows:

For topical administration to the eye, the compound represented by Formula I, a geometric isomer thereof, or a pharmaceutically acceptable salt and/or solvate and/or hydrate thereof can be made into a suitable micronized suspension or solution, the carrier used is an isotonic sterile saline with a certain pH, to which a preservative such as benzyl alkoxide chloride may or may not be added. In addition, for ophthalmic use, the compound can be made into an ointment form such as petrolatum ointment.

For topical administration to the skin, the compound represented by Formula I, a geometric isomer thereof, or a pharmaceutically acceptable salt and/or solvate and/or hydrate thereof can be made into a suitable ointment, lotion or cream preparation form, in which the active ingredient is suspended or dissolved in one or more carriers. The carriers that can be used in the ointment here include, but are not limited to: mineral oil, liquid petrolatum, white petrolatum, propylene glycol, polyethylene oxide, polypropylene oxide, emulsified wax and water; the carriers that can be used in the lotion or cream include, but are not limited to: mineral oil, sorbitan monostearate, Tween 60, cetyl ester wax, hexadecene aryl alcohol, 2-octyldodecanol, benzyl alcohol and water.

For topical administration to the lower intestinal tract, the compound represented by Formula I, a geometric isomer

thereof, or a pharmaceutically acceptable salt and/or solvate and/or hydrate thereof can be made into the above-mentioned rectal suppository preparation or a suitable enema preparation form. In addition, topical transdermal patch may also be used.

The compound represented by Formula I, a geometric isomer thereof, or a pharmaceutically acceptable salt and/or solvate and/or hydrate thereof can also be administered in the form of sterile injectable preparation, including sterile injectable water or oil suspension, or sterile injectable solution. Among them, the carrier or solvent that can be used are water, Ringer's solution and isotonic sodium chloride solution. In addition, sterile non-volatile oil such as mono- or diglyceride can also be used as a solvent or suspending medium.

The drugs for the above-mentioned various dosage forms can be prepared according to the conventional methods in the pharmaceutical field.

As used herein, a "therapeutically effective amount" or "prophylactically effective amount" refers to an amount sufficient to treat or prevent a patient's disease but low enough to avoid serious side effects (at a reasonable benefit/risk ratio) within the scope of reasonable medical judgment. The therapeutically effective amount of a compound depends on the specific compound selected (e.g., taking the efficacy, effectiveness and half-life of the compound into account), the route of administration selected, the disease being treated, the severity of the disease being treated, the age, size, weight and physical disease of the patient being treated, the medical history of the patient being treated, the duration of treatment, the nature of concurrent therapy, the desired therapeutic effect, and the like, but can still be routinely determined by those skilled in the art.

In addition, it should be pointed out that the specific dosage and usage of the compound represented by Formula I, a geometric isomer thereof, or a pharmaceutically acceptable salt and/or solvate and/or hydrate thereof for different patients are determined by many factors, including the patient's age, weight, gender, natural health status, nutritional status, active strength of the drug, time of administration, metabolic rate, severity of the condition, and the subjective judgment of the physician who makes diagnosis and treatment. It is preferred here to use a dose between 0.001 and 1000 mg/kg body weight/day.

## Brief Description of the Drawings

Figure 1 shows the dose-response curves of tetrandrine, remdesivir and chloroquine phosphate for inhibition of SARS-CoV-2 in Example 1, wherein the blue dots represent the cytotoxicity percentages of the drug at different concentrations; the red curve represents the inhibition percentages of virus yield of the drug at different concentrations, the multiplicity of infection (MOI) of virus for the upper figure is 0.01; the multiplicity of infection (MOI) of virus for the lower figure is 0.05.

Figure 2 shows the dose-response curves of tetrandrine, remdesivir and chloroquine phosphate for inhibition of SARS-CoV-2 in Example 2, wherein the blue dots represent the cytotoxicity percentages of the drug at different concentrations; the red curve represents the inhibition percentages of virus yield of the drug at different concentrations, the multiplicity of infection (MOI) of virus for the upper figure is 0.01; the multiplicity of infection (MOI) of virus for the lower figure is 0.05.

Figure 3 shows the quantitative RT-PCR detection results of tetrandrine for inhibition of SARS-CoV-2 virus infection on Vero cells.

## Specific Models for Carrying Out the present invention

The substantive contents of the present application will be further described below with reference to the specific examples of the present application. It should be understood that the following examples are only used to illustrate the present application, but are not intended to limit the protection scope of the present application. If the specific conditions are not indicated in the following examples, they are carried out according to the conventional conditions or the manufacturer's suggestion. The medicines or reagents used without the manufacturer's indication are conventional products that can be obtained from the market.

While many of the materials and operation methods used in the following examples are known in the art, they are still described in the present application as much detail as possible. It is clear to those skilled in the art that the materials and operating methods used in the following examples are well known in the art unless otherwise specified.

**Example 1: Pharmacodynamic study 1 of tetrandrine on SARS-CoV-2**

1. Materials and methods

1.1 Drugs: Tetrandrine was obtained from Zhejiang Jinhua CONBA Pharmaceutical Co., Ltd., the batch number of which is YG1910005, the content of which is 99.3%. The reference drug, chloroquine phosphate, was purchased from Sigma company, Cat. No. C6628; Remdesivir was purchased from MedChemExpresss company, Cat. No. HY-104077. Tetrandrine was prepared into 10 mM stock solution with DMSO, remdesivir was prepared into 100 mM stock solution with DMSO, and chloroquine phosphate was prepared into 100 mM stock solution with PBS. During the experiment, they were diluted with MEM medium containing 2% fetal bovine serum to the concentrations required for the experiment.
1.2 Cells: Vero-E6 cells, purchased from ATCC, Cat. No. 1586.
1.3 Virus: SARS-CoV-2 virus (nCoV-2019BetaCoV/Wuhan/WIV04/2019 isolate, the GISAID accession number was EPI_1SL_402124) was isolated and subcultured by Wuhan Institute of Virology, Chinese Academy of Sciences. During the experiment, the virus was diluted to the concentration required for the experiment with MEM medium containing 2% fetal bovine serum.
1.4. Reagents, laboratory supplies and instruments:
1.4.1 Reagents: MEM dry powder, purchased from GIBCO, USA, Cat. No. 10370021; fetal bovine serum (FBS), purchased from GIBCO, USA, Cat. No. 16000044; sodium bicarbonate, purchased from Sinopharm; penicillin, streptomycin and kanamycin: all were purchased from North China Pharmaceutical Company Ltd.; PBS, purchased from GIBCO, Cat. No. C10010500BT.
1.4.2 Experimental supplies and instruments: culture flask, purchased from Corning Company, USA; 96-well culture plate, purchased from Corning Company, USA; carbon dioxide incubator, purchased from Thermo Company, USA;
1.4.3 Preparation of cell culture medium and reagents:

The cell culture medium was MEM medium, per 100ml of which contained 10% fetal bovine serum, penicillin, streptomycin and kanamycin each 100U/ml, and 5% NaHCOs.

Cell digestion solution: 0.25% trypsin, prepared with Hanks solution, 0.02% EDTA.

1.5. Experimental method:

1.5.1 Vero E6 cell culture: 0.1 ml of 0.25% trypsin, 5 ml of 0.02% EDTA were added to a culture flask full of cells, the digestion was carried out at 37°C for 5 minutes, then the digestion solution was discarded, the cell culture solution was added and then mixed by pipetting up-down. The cells were subcultured at 1:3, reached confluence within 3 days, formulated into 100,000 cells per ml, inoculated to a 96-well cell culture plate, 0.1 ml per well, and cultured at 37°C, 5% $CO_2$ for 24 hours, and the cells grew into monolayer was used for experiment.

1.5.2 Experiment of drug cytotoxicity on cells:
The detection of drug cytotoxicity was determined using CellTiter-Glo kit (Promega). Specific steps were as follows:

① $1\times10^4$ Vero-E6 cells were inoculated in a 96-well plate and cultured at 37°C for 8 hours.

② The stock solution of drug was diluted with MEM medium containing 2% FBS to the desired concentration, the original medium in the 96-well plate was discarded, and 100 μL of the drug-containing MEM medium was taken and added to the corresponding wells, so that the final concentrations of drug in the wells were those shown in Tables 1 to 3, respectively, and three replicate wells were made for each concentration. At the same time, a vehicle control (adding DMSO or PBS, and 2% FBS-containing MEM medium without drug to the cell wells) and a blank control (adding DMSO and 2% FBS-containing MEM medium to the wells without cells) were set. After the addition of drug, the cells were incubated at 37°C for 24 hours.

③ 100 μL of CellTiter-Glo solution (Promega) was added to the wells to be tested, and mixed by shaking for 2 min to fully lyse the cells, incubated at room temperature for 10 minutes, and readings of chemiluminescence signals OD450 were obtained on a microplate reader (purchased from Molecular Devices, model: SpectraMax M5). The cell viability was calculated by taking the readings into the following formula:

$$\text{Cell viability (\%)} = (A_{\text{(drug treatment group)}} - A_{\text{(blank control)}}) / (A_{\text{(vehicle control)}} - A_{\text{(blank control)}}) \times 100\%$$

where A represented the reading on the microplate reader.

1.5.3 Antivirus experiment:

① Drug treatment
Vero E6 cells were inoculated into a 48-well plate, about $1\times10^5$ cells per well, and the experiment was performed on the next day. Firstly, 100 μL of MEM medium (containing 2% FBS) containing the corresponding concentration of drug was added to the plate, the cells were pretreated for 1 hour, then 20 μL of diluted virus (the amount of virus was 1000 $TCID_{50}$, that was, MOI=0.01, and the amount of virus was 5000 $TCID_{50}$, that was, MOI=0.05) was added, and then the cells were placed in an incubator and incubated for 1 hour. Then the virus culture medium was discarded, the uninfected residual virus was washed away with PBS, and then MEM culture media (containing 2% FBS) that contained the corresponding concentrations of tetrandrine, remdesivir and chloroquine phosphate were added, respectively, so that the final concentrations of drug in the wells were those shown in Table 4 to Table 6, respectively, and then the cells was placed into a 37°C, 5% $CO_2$ incubator and subsequently cultured for 48h. To the cell control group, a final concentration of 0.3% DMSO in MEM culture medium containing 2% FBS or 0.5% PBS in MEM medium containing 2% FBS was added.

(2) RNA extraction
RNA extraction was performed using a kit produced by TaKaRa company (TaKaRa MiniBEST Viral RNA/DNA Extraction Kit, Cat. No. 9766). Unless otherwise specified, the consumables and reagents involved in the following RNA extraction steps were parts of the kit. The following extraction steps were all recommended in the kit instruction.

1) 100 μL of the supernatant was taken from the test plate, added to a nuclease-free EP tube (purchased from Axygen, Cat. No. mct-150-c), then 321 μL of lysis solution (100 μL PBS, 200 μL buffer VGB, 2μL proteinase K, 1μL carrier RNA) was added to per well, mixed well, and then digestion was carried out at 56°C for 15 min;

2) 200 μL of absolute ethanol was added to the mixture obtained in step 1), and mixed well;

3) the mixture obtained in step 2) was transferred into an RNase-free spin column, centrifuged at 12,000 rpm for 15s, and the waste liquid was discarded;

4) 500 μL of Buffer RW1 was added, centrifuged at 12,000 rpm for 15s to wash the spin column, and the waste liquid was discarded;

5) 650μL of Buffer RW2 was added, centrifuged at 12000 rpm for 15s to wash the spin column, and the waste liquid was discarded;

6) 650 μL of Buffer RW2 was added, centrifuged at 12,000 rpm for 2 min to wash the spin column, the waste liquid was discarded, and then the entire spin column was transferred to a new RNase-free 2 ml collection tube in step 7);

7) a new RNase-free 2ml collection tube was used for exchange, centrifugation was carried out at 12,000 rpm for 1 min, the spin column was dried, and then the entire spin column was transferred to a 1.5ml collection tube in step 8);

8) a new 1.5 ml collection tube was used for exchange, the dried spin column in step 7) was placed therein, 30 μL of RNase-free water was added to each spin column, centrifugation was carried out at 12,000 rpm for 2 min, and the resultant eluate contained the corresponding RNA.

③ RNA reverse transcription
A reverse transcription kit (PrimeScript™ RT reagent Kit with gDNA Eraser, Cat. No. RR047Q) produced by TaKaRa company was used for RNA reverse transcription. The steps were as follows:

1) Removal of gDNA: RNA samples were collected from each experimental group, and 3 μL of RNA was taken for reverse transcription. First, 2 μL of 5× gDNA Eraser Buffer was added to the RNA sample of each experimental group, the reaction system was supplemented to 10 μL with RNase-free water, mixed well,

and subjected to water bath at 42°C for 2 min to remove the gDNA that might be present in the sample;

2) Reverse transcription: Appropriate amounts of enzyme, primer Mix and reaction buffer were added to the sample obtained in step 1), RNase-free water was added to supplement to a volume of 20 μL, the reaction was performed in a 37°C water bath for 15 minutes, and then in a 85°C water bath for 5 sec, to obtain cDNA by transcription.

④ Real-time PCR.

Fluorescence quantitative PCR was used to detect the copy number per milliliter of the original virus solution.

The reaction system was mixed with TB Green Premix (Takara, Cat#RR820A), and the amplification reaction and reading were performed with a StepOne Plus Real-time PCR instrument (brand: ABI). The copy number per milliliter of the original virus solution was calculated. The steps were as follows:

1) First, standard product were established. Plasmid pMT-RBD was diluted into $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL, respectively. 2 μL of standard product or cDNA template was taken for qPCR reaction;

2) The primer sequences used in the experiment were as follows (all were indicated in the 5'-3' direction):

RBD-qF: CAATGGTTTAACAGGCACAGG

RBD-qR: CTCAAGTGTCTGTGGATCACG

3) The reaction procedure was as follows:

Pre-denaturation: 95°C for 5 minutes;

Cycling parameters: 95°C for 15 seconds, 54°C for 15 seconds, 72°C for 30 seconds. 40 cycles in total.

2. Results

2.1. Toxicity of tetrandrine, chloroquine phosphate and remdesivir on Vero E6 cells

The cytotoxicity results showed that the treatment of the test compounds did not change the cell viability at all the tested concentrations, that was, the test compounds had no toxic effect on the cells at all concentrations (Tables 1 to 3).

Table 1. Cytotoxicity experiment of test compound tetrandrine

| | Tetrandrine | | | | | | | |
|---|---|---|---|---|---|---|---|---|
| Concentration (μM) | 100 | 50 | 25 | 12.5 | 6.25 | 3.13 | 1.56 | Vehicle |
| Cell viability (% of vehicle control) | 0.96±0.14 | 0.28±0.32 | 58.25±2.25 | 78.14±1.98 | 91.24±1.55 | 97.38±1.04 | 94.81±2.84 | 100.00±4.36 |

Table 2. Cytotoxicity experiment of test compound remdesivir

| Remdesivir | | | | | | | | |
|---|---|---|---|---|---|---|---|---|
| Concentration (μM) | 200 | 100 | 50 | 25 | 12.5 | 6.25 | 3.13 | Vehicle |
| Cell viability (% of vehicle control) | 40.05±1.33 | 70.09±0.62 | 90.20±4.90 | 95.71±0.82 | 99.62±3.00 | 99.17±2.36 | 97.16±2.53 | 100.00±0.93 |

Table 3. Cytotoxicity experiment of test compound chloroquine phosphate

| Chloroquine phosphate | | | | | | | | |
|---|---|---|---|---|---|---|---|---|
| Concentration (μM) | 300 | 200 | 100 | 66.67 | 33.33 | 11.11 | 3.70 | Vehicle |
| Cell viability (% of vehicle control) | 0.63±0.30 | 41.01±1.95 | 44.84±1.79 | 68.35±1.16 | 72.67±2.95 | 90.60±3.49 | 93.26±2.52 | 100.00±1.64 |

2.2. Antiviral activity of tetrandrine, chloroquine phosphate and remdesivir

The results of virus proliferation inhibition experiment showed that the test compounds at different concentrations could effectively inhibit the replication of viral genome in the infected cell supernatant by SARS-CoV-2 (Tables 4 to 6 and Figure 1).

Table 4. Antiviral experiment of test compound tetrandrine

| | Tetrandrine | | | | | | |
|---|---|---|---|---|---|---|---|
| Concentration (μM) | 20 | 10 | 5 | 2.5 | 1.25 | 0.625 | Vehicle |
| Copy number of viral genome (MOI = 0.01) | 1323393±14 8300 | 1456943±42 494 | 373861479± 310383208 | 1707286250 ±252757047 | 3336005167 ±635129185 | 3357508000 ±476420774 | 4646163000 ±630749778 |
| Copy number of viral genome (MOI = 0.05) | 4092752±17 07243 | 8161310±19 70471 | 2477488500 ±123478263 6 | 7118504000 ±142756291 7 | 7198143667 ±574130625 | 1066955866 7±21242666 71 | 1050018900 0±90694959 3 |

Table 5. Antiviral experiment of test compound remdesivir

| Remdesivir | | | | | | | |
|---|---|---|---|---|---|---|---|
| Concentration (μM) | 30 | 10 | 3.33 | 1.11 | 0.37 | 0.12 | Vehicle |
| Copy number of viral genome (MOI = 0.01) | 975616± 267388 | 563645±305 066 | 243294±396 633 | 19898611±4 384275 | 1901120292 ±579712007 | 3900176000 ±567399802 | 4646163000 ±630749778 |
| Copy number of viral genome (MOI = 0.05) | 2594526±32 6599 | 3230111±18 37657 | 13188656±2 14715 | 545157271± 238234317 | 9405568167 ±193769423 3 | 1159417500 0±27546388 36 | 1050018900 0±90694959 3 |

Table 6. Antiviral experiment of test compound chloroquine phosphate

| | Chloroquine phosphate | | | | | | |
|---|---|---|---|---|---|---|---|
| Concentration (μM) | 50 | 16.67 | 5.56 | 1.85 | 0.62 | 0.21 | Vehicle |
| Copy number of viral genome (MOI = 0.01) | 1019575±26 4173 | 11907226±3 553865 | 692991541± 155454354 | 1500455250 ±191634910 | 2407772083 ±85393605 | 3451032833 ±662740949 | 3393134375 ±303897692 |
| Copy number of viral genome (MOI = 0.05) | 1914748±14 6170 | 52973263±2 5725143 | 1814432458 ±127478253 | 4521430250 ±566280195 | 5663562000 ±672504403 | 5871676583 ±157934682 | 5652325333 ±111931351 7 |

After calculation, under the condition of virus multiplicity of infection (MOI) of 0.01:

Tetrandrine: $EC_{50}$ = 1.71 μM, $CC_{50}$ = 24.51 μM, SI = 14.33

Remdesivir: $EC_{50}$ = 0.30 μM, $CC_{50}$ = 160.30 μM, SI = 534.33

Chloroquine phosphate: $EC_{50}$ = 1.54 μM, $CC_{50}$ = 92.93 μM, SI = 60.34

Under the condition of virus multiplicity of infection (MOI) of 0.05:

Tetrandrine: $EC_{50}$ = 2.88 μM, $CC_{50}$ = 24.51 μM, SI = 8.51

Remdesivir: $EC_{50}$ = 0.59 μM, $CC_{50}$ = 160.30 μM, SI = 271.69

Chloroquine phosphate: $EC_{50}$ = 3.77 μM, $CC_{50}$ = 92.93 μM, SI = 24.49

3. Conclusion

Tetrandrine (the reference substances are chloroquine phosphate and remdesivir) had good safety for the test cells in vitro at different test concentrations. Tetrandrine, remdesivir and chloroquine phosphate had obvious inhibitory effects against the SARS-CoV-2 virus isolate nCoV-2019BetaCoV/Wuhan/WIV04/2019, and when the MOI of virus infection was 0.01, the $EC_{50}$ values of which were 1.71 μM, 0.30 μM and 1.54 μM, respectively, and the corresponding selectivity index of which were 14.33, 534.33 and 60.34, respectively, when the MOI of virus infection was 0.05, the $EC_{50}$ values of which were 2.88 μM, 0.59 μM and 3.77 μM, respectively, and the corresponding selectivity index of which were 8.51, 271.69 and 24.49, respectively.

Tetrandrine, chloroquine phosphate and remdesivir had inhibitory activity against SARS-CoV-2 virus in vitro.

**Example 2: Pharmacodynamic study 2 of tetrandrine on SARS-CoV-2**

The experimental materials and methods were the same as those in Example 1. The specific difference was that in the antiviral experiment, the drug treatment step in Example 1 comprised first adding 100 μL of MEM culture medium (containing 2% FBS) containing the corresponding concentration of drug to the plate, and the cells were pretreated for 1 hour, while in this example, the drug treatment step did not comprise pretreating cells with drug, and the specific steps were as follows:
Vero E6 cells were inoculated into a 48-well plate, about $1\times10^5$ cells per well, and then 20 μL of diluted virus was added (the amount of virus was 1000 $TCID_{50}$, that was, MOI=0.01, and the amount of virus was 5000 $TCID_{50}$, that was, MOI=0.05), and then the cells were placed in an incubator and incubated for 1 hour. After 1 h, the virus culture medium was discarded, the uninfected residual virus was washed away with PBS, and MEM culture media (containing 2% FBS) containing the corresponding concentrations of tetrandrine, remdesivir and chloroquine phosphate were added, respectively, so that the final concentrations of drug in the cells were the same as those drug concentrations shown in Tables 4, 5 and 6 of Example 1, then the cells was placed into a 37°C, 5% $CO_2$ incubator and subsequently cultured for 48h. To the cell control group, a final concentration of 0.3% DMSO in cell culture medium containing 2% FBS or 0.5% PBS in cell medium containing 2% FBS was added.

The methods of the subsequent RNA extraction, RNA reverse transcription and Real-time PCR were the same as in Example 1.

After calculation, under the condition of virus multiplicity of infection (MOI) of 0.01:

Tetrandrine: $EC_{50}$ = 1.61 μM, $CC_{50}$ = 24.51 μM, SI = 15.22

Remdesivir: $EC_{50}$ = 0.23 μM, $CC_{50}$ = 160.30 μM, SI =696.96

Chloroquine phosphate: $EC_{50}$ = 4.52 μM, $CC_{50}$ = 92.93 μM, SI = 20.56

Under the condition of virus multiplicity of infection (MOI) of 0.05:

Tetrandrine: $EC_{50}$ = 1.95 μM, $CC_{50}$ = 24.51 μM, SI = 12.51

Remdesivir: $EC_{50}$ =0.62 μM, $CC_{50}$ = 160.30 μM, SI =258.55

Chloroquine phosphate: $EC_{50}$ = 3.63 μM, $CC_{50}$ = 92.93 μM, SI = 25.60

Conclusion:
The results of antiviral experiments showed that tetrandrine, remdesivir and chloroquine phosphate had obvious inhibitory effects against the SARS-Cov-2 virus isolate nCoV-2019BetaCoV/Wuhan/WIV04/2019, when the MOI of virus infection was 0.01, the $EC_{50}$ values of which were 1.61 μM, 0.23 μM and 4.52 μM, respectively, and the corresponding selectivity index of which were 15.22, 696.96, and 20.56, respectively; when the MOI of virus infection was 0.05, the $EC_{50}$ values of which were 1.95 μM, 0.62 μM and 3.63 μM, respectively, and the corresponding selectivity index of which were 12.51, 258.55 and 25.60, respectively. (See Figure 2)

Tetrandrine, chloroquine phosphate and remdesivir had inhibitory activity against SARS-Cov-2 virus after virus infection of cells.

**Example 3: Half-maximal effective concentration ($EC_{50}$) of tetrandrine against SARS-CoV-2**

The $EC_{50}$ of the drug was determined by nucleic acid quantification. Specifical steps were as follows: Vero cells were inoculated into a 48-well plate at a concentration of about 10,000 cells/well one day in advance. The drug tetrandrine was prepared to a final concentration of 20, 10, 5, 2.5, 1.25, 0.625 and 0.3125 μM with DMEM medium containing 2% FBS (purchased from Gibco, Cat. No. 16000044), and added to the cells, the cells were placed in a 37°C, 5% $CO_2$ incubator to be pretreated for 1 hour. Then, SARS-CoV-2 virus (nCoV-2019BetaCoV/Wuhan/WIV04/2019 isolate, the GISAID accession number was EPI_ISL_402124, isolated and subcultured by Wuhan Institute of Virology, Chinese Academy of Sciences) was diluted with DMEM medium containing 2% FBS, and added to the corresponding wells to make the viral load as 100 $TCID_{50}$, subjected to adsorption and culture at 37°C for 2 hours, then the virus solution was discarded, and tetrandrine at different concentrations was added to each well (200 μL/well) so that the final concentrations of tetrandrine were 20, 10, 5, 2.5, 1.25, 0.625 and 0.3125 μM in the well, 3 duplicate wells were set for each drug concentration, and a virus control group and a normal cell control group were set up, the culture was performed in a 37°C, 5% $CO_2$ incubator, and cytopathic effects (CPE) were observed daily. Two days after infection, 50 μL of cell supernatant was taken from each well to extract nucleic acid, and the viral load was detected by quantitative RT-PCR.

The methods of RNA extraction, RNA reverse transcription and RT-PCR were the same as in Example 1.

According to the conversion formula of viral RNA copy number and CT value: RNA Copies/mL = CT*(-0.3) + 13.17, the viral load was calculated. The formula: (infection rate (%) = RNA copy number of drug group / RNA copy number of virus control group × 100%) was used to calculate the infection rate (%) of virus at different concentrations of drug treatment, and Graphpad Prism 7 software was used to perform S-fitting analysis on the data, and the fitting results were shown in Figure 3, and $EC_{50}$ was calculated.

The results showed that tetrandrine had an inhibitory effect against the SARS-CoV-2 virus at the cellular level with an $EC_{50}$ of 8.99 μM (Figure 3).

Although the specific embodiments of the present application have been described in detail, those skilled in the art will understand that, based on all the teachings disclosed, various modifications and substitutions of those details may be made, and these changes are all within the protection scope of the present application. The full scope of the present application is given by the appended claims and any equivalents thereof.

**References:**

Zaki AM, van Boheemen S, Bestebroer T M, et al. Isolation of a novel coronavirus from a man with pneumonia in Saudi Arabia[J]. N Engl J Med, 2012, 367(19): 1814-1820.

Zhong NS, Zheng BJ, Li YM, et al. Epidemiology and cause of severe acute respiratory syndrome (SARS) in Guangdong, People's Republic of China, in February, 2003[J]. The Lancet, 2003, 362(9393): 1353 -1358.

Cui J, Li F, Shi ZL. Origin and evolution of pathogenic coronaviruses[J]. Nat Rev Microbiol, 2019, 17(3): 181-192.

Wang M, Cao R, Zhang L, et al. Remdesivir and chloroquine effectively inhibit the recently emerged novel coronavirus (2019-nCoV) in vitro[J]. Cell Res, 2020, 0: 1-3.

## Claims

1. Use of a compound represented by Formula I, a geometric isomer thereof or a pharmaceutically acceptable salt and/or solvate and/or hydrate thereof,

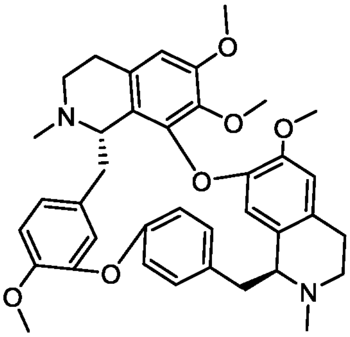

I

or a pharmaceutical composition comprising a compound represented by Formula I, a geometric isomer thereof or a pharmaceutically acceptable salt and/or solvate and/or hydrate thereof in the manufacture of a product for the prevention and/or treatment of a disease or infection caused by a coronavirus or a disease or infection caused by a coronavirus and accompanied with hypertension.

2. Use of a compound represented by Formula I, a geometric isomer thereof or a pharmaceutically acceptable salt and/or solvate and/or hydrate thereof,

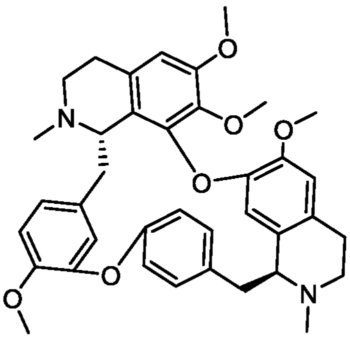

I

or a pharmaceutical composition comprising a compound represented by Formula I, a geometric isomer thereof or a pharmaceutically acceptable salt and/or solvate and/or hydrate thereof in the manufacture of a product for the prevention and/or treatment of a pulmonary disease or symptom associated with a coronavirus and accompanied with hypertension.

3. Use of a compound represented by Formula I, a geometric isomer thereof or a pharmaceutically acceptable salt and/or solvate and/or hydrate thereof,

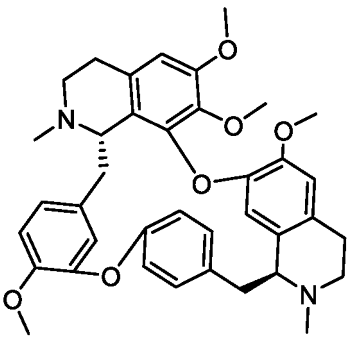

I

or a pharmaceutical composition comprising a compound represented by Formula I, a geometric isomer thereof or a pharmaceutically acceptable salt and/or solvate and/or hydrate thereof in the manufacture of a product for inhibiting the replication or reproduction of a coronavirus in a cell (e.g., a cell of mammal), or in the manufacture of a product as a coronavirus inhibitor.

**4.** Use of a compound represented by Formula I, a geometric isomer thereof or a pharmaceutically acceptable salt and/or solvate and/or hydrate thereof,

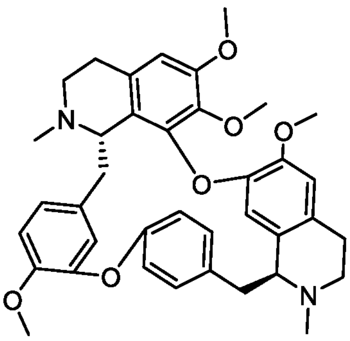

I

or a pharmaceutical composition comprising a compound represented by Formula I, a geometric isomer thereof or a pharmaceutically acceptable salt and/or solvate and/or hydrate thereof in the manufacture of a product for the prevention and/or treatment of a pulmonary disease or symptom associated with SARS-CoV-2 or an asymptomatic or symptomatic SARS-CoV-2 infection.

**5.** Use of a compound represented by Formula I, a geometric isomer thereof or a pharmaceutically acceptable salt and/or solvate and/or hydrate thereof,

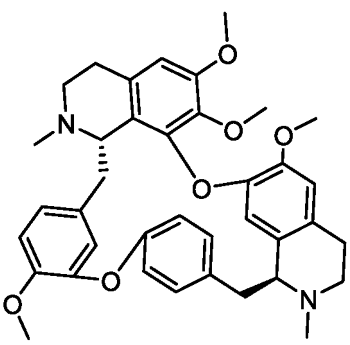

I

or a pharmaceutical composition comprising a compound represented by Formula I, a geometric isomer thereof or a pharmaceutically acceptable salt and/or solvate and/or hydrate thereof in the manufacture of a product for the prevention and/or treatment of a pulmonary disease or symptom associated with SARS-CoV-2 and accompanied with hypertension or an asymptomatic or symptomatic SARS-CoV-2 infection accompanied with hypertension.

**6.** Use of a compound represented by Formula I, a geometric isomer thereof or a pharmaceutically acceptable salt and/or solvate and/or hydrate thereof,

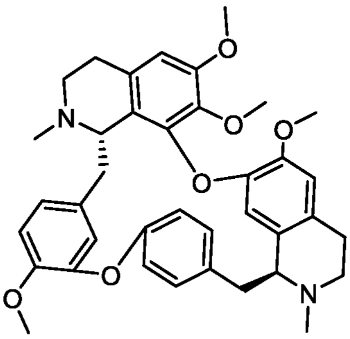

I

or a pharmaceutical composition comprising a compound represented by Formula I, a geometric isomer thereof or a pharmaceutically acceptable salt and/or solvate and/or hydrate thereof in the manufacture of a product for the prevention and/or treatment of a COVID-19 or a COVID-19 accompanied with hypertension, or in the manufacture of a product as a SARS-CoV-2 inhibitor, or in the manufacture of a product for inhibiting the replication or reproduction of SARS-CoV-2 in a cell (e.g., a cell of mammal).

**7.** The use according to any one of claims 1 to 6, wherein the product is a human drug or a veterinary drug for animal.

**8.** The use according to claim 7, wherein the product is a solid preparation, injection, spray, liquid preparation, inhalation preparation or compound preparation.

**9.** The use according to claim 3 or 6, wherein the mammal comprises bovine, equine, caprinae, suidae, canine, feline, rodent, primate, for example, it is a human, dog or pig.

**10.** The use according to any one of claims 1 to 6, wherein the pharmaceutical composition further comprises a pharmaceutically acceptable carrier or excipient, specifically, the pharmaceutical composition is a solid preparation, injection, inhalation preparation, spray, liquid preparation, or compound preparation.

**11.** A compound represented by Formula I, a geometric isomer thereof or a pharmaceutically acceptable salt and/or solvate and/or hydrate thereof,

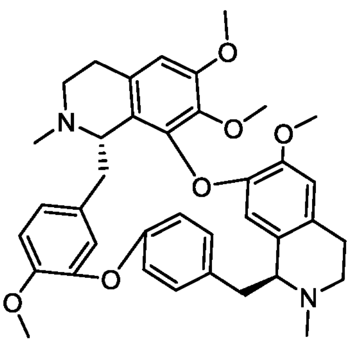

I

or a pharmaceutical composition comprising a compound represented by Formula I, a geometric isomer thereof or a geometric isomer thereof or

a pharmaceutically acceptable salt and/or solvate and/or hydrate thereof, for use in the prevention and/or treatment of a disease or infection caused by a coronavirus.

**12.** A compound represented by Formula I, a geometric isomer thereof or a pharmaceutically acceptable salt and/or solvate and/or hydrate thereof,

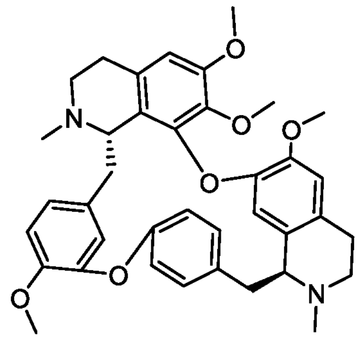

I

or a pharmaceutical composition comprising a compound represented by Formula I, a geometric isomer thereof or a pharmaceutically acceptable salt and/or solvate and/or hydrate thereof, for use in the prevention and/or treatment of a pulmonary disease or symptom associated with a coronavirus and accompanied with hypertension.

**13.** A compound represented by Formula I, a geometric isomer thereof or a pharmaceutically acceptable salt and/or solvate and/or hydrate thereof,

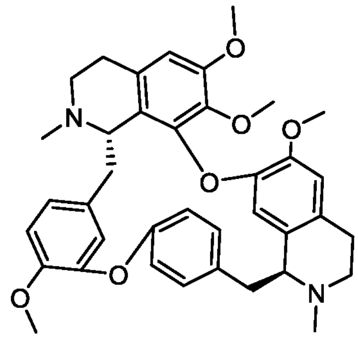

I

or a pharmaceutical composition comprising a compound represented by Formula I, a geometric isomer thereof or a pharmaceutically acceptable salt and/or solvate and/or hydrate thereof, for use in inhibiting the replication or reproduction of a coronavirus in a cell (e.g., a cell of mammal).

**14.** A compound represented by Formula I, a geometric isomer thereof or a pharmaceutically acceptable salt and/or solvate and/or hydrate thereof,

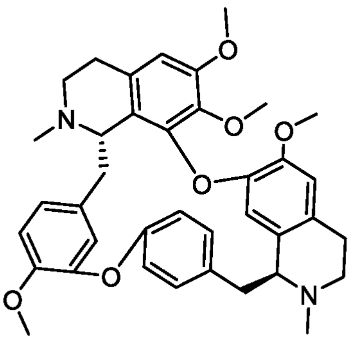

I

or a pharmaceutical composition comprising a compound represented by Formula I, a geometric isomer thereof or a pharmaceutically acceptable salt and/or solvate and/or hydrate thereof, for use as a coronavirus inhibitor.

**15.** A compound represented by Formula I, a geometric isomer thereof or a pharmaceutically acceptable salt and/or solvate and/or hydrate thereof,

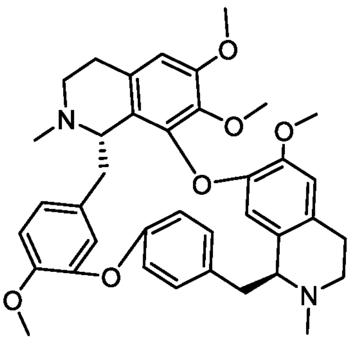

I

or a pharmaceutical composition comprising a compound represented by Formula I, a geometric isomer thereof or a pharmaceutically acceptable salt and/or solvate and/or hydrate thereof, for use in the prevention and/or treatment of a pulmonary disease or symptom associated with SARS-CoV-2 or an asymptomatic or symptomatic SARS-CoV-2 infection.

**16.** A compound represented by Formula I, a geometric isomer thereof or a pharmaceutically acceptable salt and/or solvate and/or hydrate thereof,

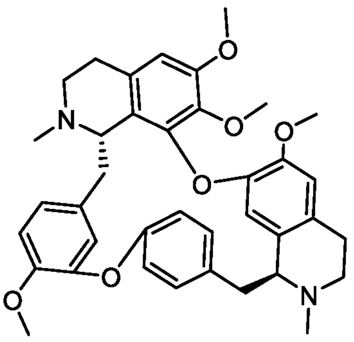

I

or a pharmaceutical composition comprising a compound represented by Formula I, a geometric isomer thereof or a pharmaceutically acceptable salt and/or solvate and/or hydrate thereof, for use in the prevention and/or treatment

of a pulmonary disease or symptom associated with SARS-CoV-2 and accompanied with hypertension or an asymptomatic or symptomatic SARS-CoV-2 infection accompanied with hypertension.

**17.** A compound represented by Formula I, a geometric isomer thereof or a pharmaceutically acceptable salt and/or solvate and/or hydrate thereof,

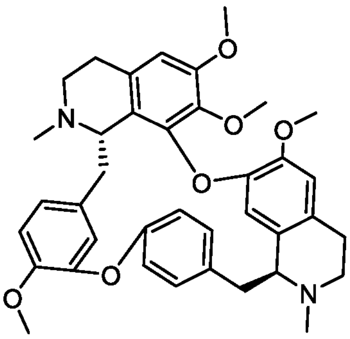

I

or a pharmaceutical composition comprising a compound represented by Formula I, a geometric isomer thereof or a pharmaceutically acceptable salt and/or solvate and/or hydrate thereof, for use in the prevention and/or treatment of a COVID-19 or a COVID-19 accompanied with hypertension.

**18.** A compound represented by Formula I, a geometric isomer thereof or a pharmaceutically acceptable salt and/or solvate and/or hydrate thereof,

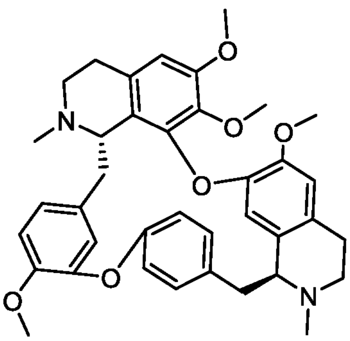

I

or a pharmaceutical composition comprising a compound represented by Formula I, a geometric isomer thereof or a pharmaceutically acceptable salt and/or solvate and/or hydrate thereof, for use as a SARS-CoV-2 inhibitor.

**19.** A compound represented by Formula I, a geometric isomer thereof or a pharmaceutically acceptable salt and/or solvate and/or hydrate thereof,

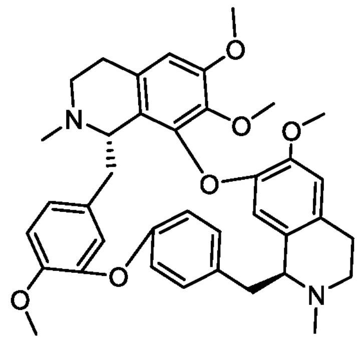

I

or a pharmaceutical composition comprising a compound represented by Formula I, a geometric isomer thereof or a pharmaceutically acceptable salt and/or solvate and/or hydrate thereof, for use in inhibiting the replication or reproduction of SARS-CoV-2 in a cell (e.g., a cell of mammal).

**20.** A method for preventing and/or treating a disease or infection caused by a coronavirus or a disease or infection caused by a coronavirus and accompanied with hypertension, comprising administering to a host in need thereof a prophylactically and/or therapeutically effective amount of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof.

**21.** A method of preventing and/or treating a pulmonary disease or symptom associated with a coronavirus or a pulmonary disease or symptom associated with a coronavirus and accompanied with hypertension, comprising administering to a host in need thereof a prophylactically and/or therapeutically effective amount of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof.

**22.** A method for inhibiting the replication or reproduction of a coronavirus in a host in need thereof, comprising administering to the host in need thereof a prophylactically and/or therapeutically effective amount of a compound represented by Formula I, a geometric isomer thereof or a pharmaceutically acceptable salt and/or solvate and/or hydrate thereof, or a pharmaceutical composition comprising a compound represented by Formula I, a geometric isomer thereof or a pharmaceutically acceptable salt and/or solvate and/or hydrate thereof.

**23.** The method according to any one of claims 20 to 22, wherein the coronavirus is SARS-CoV-2.

**24.** The method according to claim 20, wherein the disease caused by the coronavirus is a COVID-19.

**25.** The method according to claim 20, wherein the disease caused by the coronavirus and accompanied with hypertension is a COVID-19 accompanied with hypertension.

**26.** The method according to claim 21, wherein the pulmonary disease or symptom associated with the coronavirus is a pulmonary disease or symptom associated SARS-CoV-2.

**27.** The method according to claim 21, wherein the pulmonary disease or symptom associated with a coronavirus and accompanied with hypertension is a pulmonary disease or symptom associated with SARS-CoV-2 and accompanied with hypertension.

**28.** The method according to claim 20, wherein the infection caused by the coronavirus is an asymptomatic or symptomatic SARS-CoV-2 infection.

**29.** The method according to claim 20, wherein the infection caused by the coronavirus and accompanied with hypertension is an asymptomatic or symptomatic SARS-CoV-2 infection accompanied with hypertension.

**30.** The method according to any one of claims 20 to 22, wherein the host is a mammal.

**31.** The method according to any one of claims 20 to 22, wherein the mammal comprises bovine, equine, caprinae, suidae, canine, feline, rodent, primate, wherein the mammal is preferably a human, dog or pig.

**32.** The compound, a geometric isomer thereof or a pharmaceutically acceptable salt and/or solvate and/or hydrate thereof according to claim 13 or 19, wherein the mammal comprises bovine, equine, caprinae, suidae, canine, feline, rodent, primate, for example, is a human, dog or pig.

**33.** The pharmaceutical composition according to any one of claims 11 to 19 or the method according to any one of claims 20 to 22, wherein the pharmaceutical composition further comprises a pharmaceutically acceptable carrier or excipient, specifically, the pharmaceutical composition is a solid preparation, injection, inhalation preparation, spray, liquid preparation, or compound preparation.

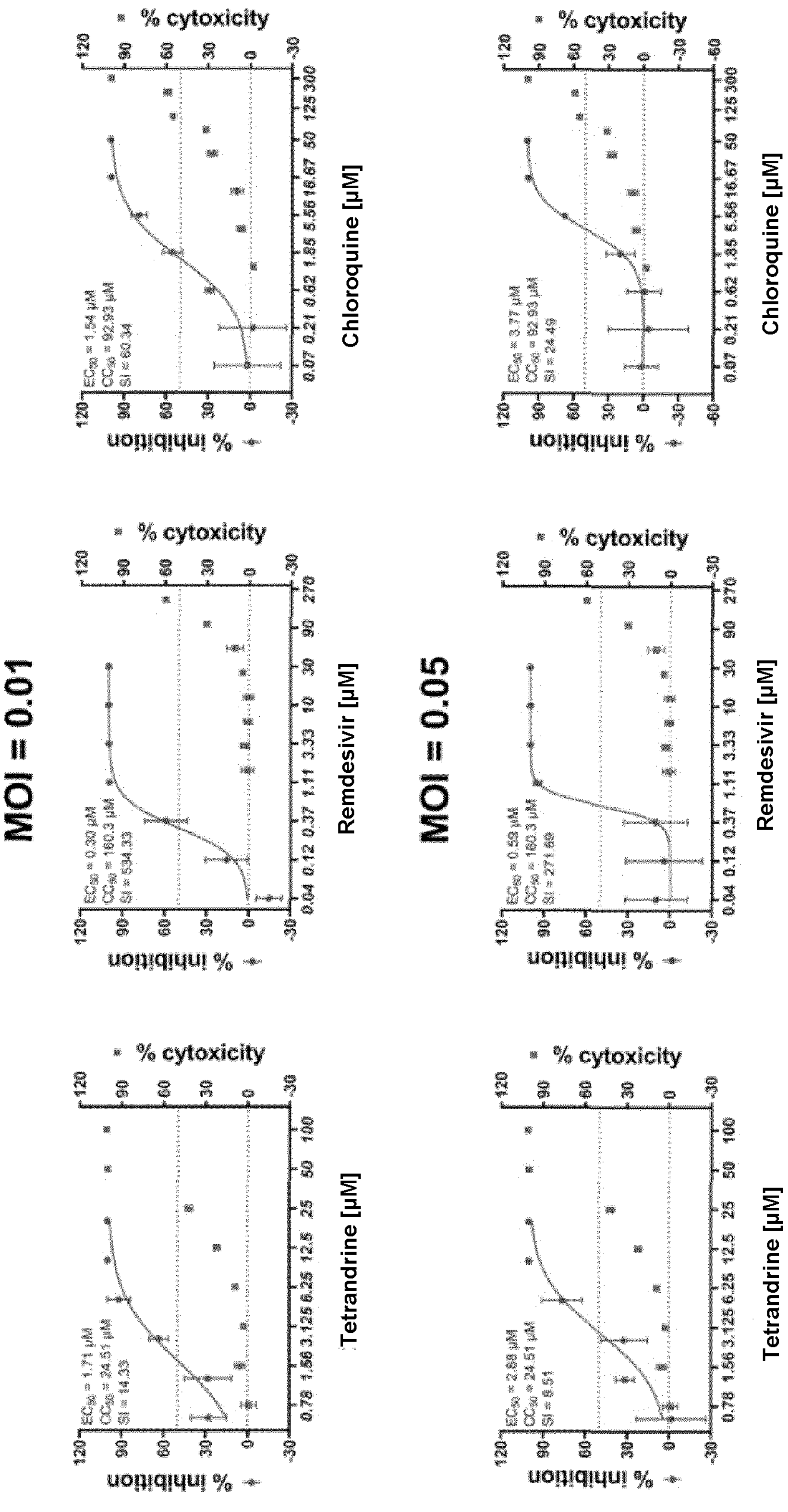
MOI = 0.01
MOI = 0.05
% inhibition
% cytoxicity
Tetrandrine [µM]
Remdesivir [µM]
Chloroquine [µM]
EC50 = 1.71 µM
CC50 = 24.51 µM
SI = 14.33
EC50 = 0.30 µM
CC50 = 160.3 µM
SI = 534.33
EC50 = 1.54 µM
CC50 = 92.93 µM
SI = 60.34
EC50 = 2.88 µM
CC50 = 24.51 µM
SI = 8.51
EC50 = 0.59 µM
CC50 = 160.3 µM
SI = 271.69
EC50 = 3.77 µM
CC50 = 92.93 µM
SI = 24.49

Figure 1

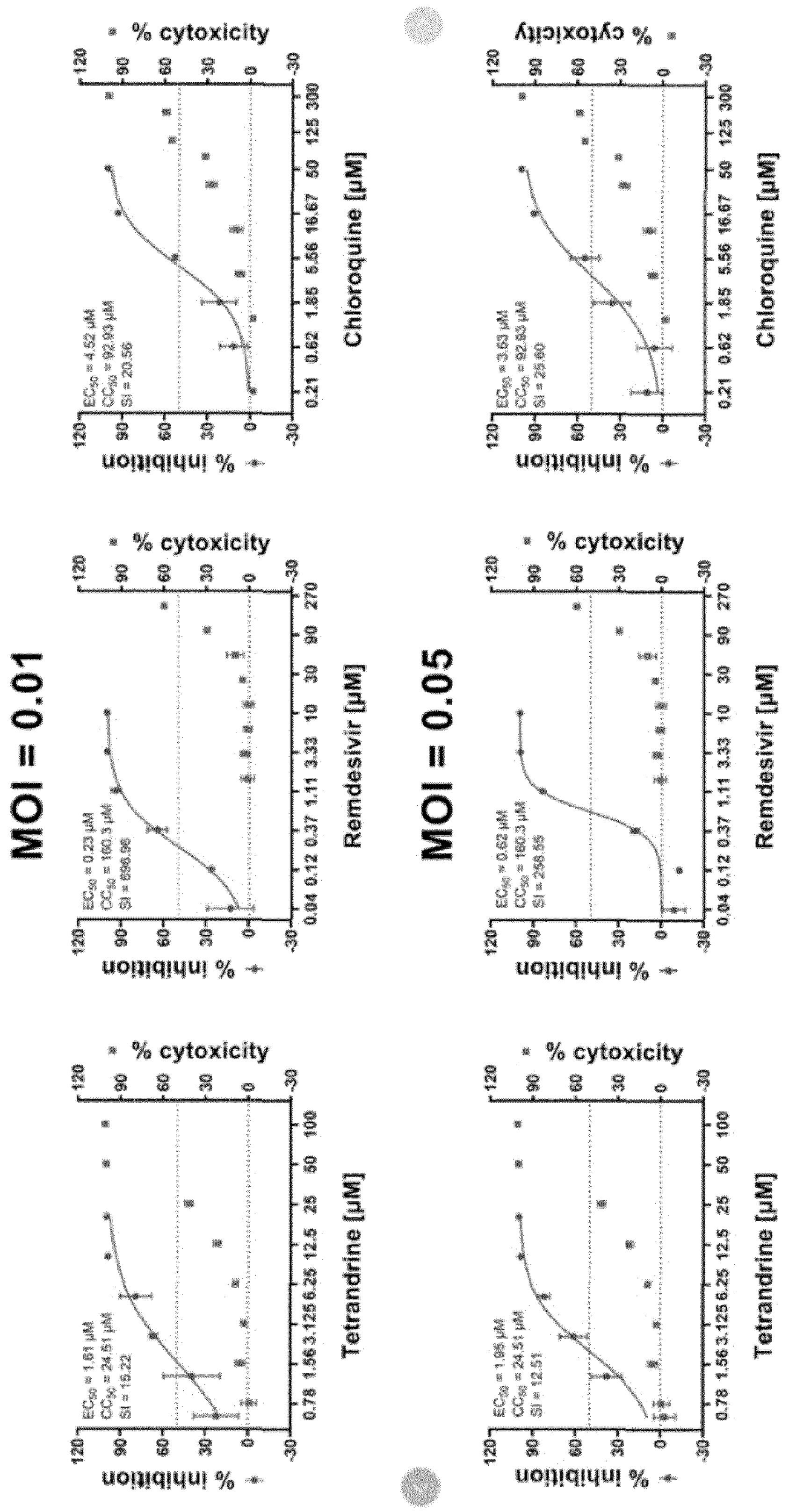
MOI = 0.01
MOI = 0.05
% inhibition
% cytoxicity
$EC_{50}$ = 1.61 µM
$CC_{50}$ = 24.51 µM
SI = 15.22
$EC_{50}$ = 1.95 µM
$CC_{50}$ = 24.51 µM
SI = 12.51
Tetrandrine [µM]
$EC_{50}$ = 0.23 µM
$CC_{50}$ = 160.3 µM
SI = 696.96
$EC_{50}$ = 0.62 µM
$CC_{50}$ = 160.3 µM
SI = 258.55
Remdesivir [µM]
$EC_{50}$ = 4.52 µM
$CC_{50}$ = 92.93 µM
SI = 20.56
$EC_{50}$ = 3.63 µM
$CC_{50}$ = 92.93 µM
SI = 25.60
Chloroquine [µM]

Figure 2

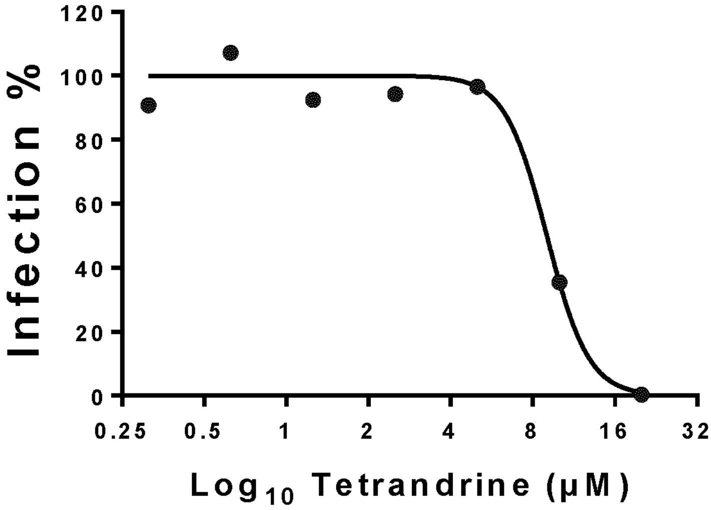
120
100
80
60
40
20
0
Infection %
0.25
0.5
1
2
4
8
16
32
Log10 Tetrandrine (µM)

Figure 3

# INTERNATIONAL SEARCH REPORT

International application No.
**PCT/CN2020/101581**

**A. CLASSIFICATION OF SUBJECT MATTER**

A61K 31/4748(2006.01)i; A61P 31/14(2006.01)i; A61P 9/12(2006.01)i; A61K 9/20(2006.01)i; A61K 9/08(2006.01)i; A61K 9/72(2006.01)i; C07D 491/18(2006.01)i; C07D 491/153(2006.01)i

According to International Patent Classification (IPC) or to both national classification and IPC

**B. FIELDS SEARCHED**

Minimum documentation searched (classification system followed by classification symbols)

A61K,A61P,C07D

Documentation searched other than minimum documentation to the extent that such documents are included in the fields searched

Electronic data base consulted during the international search (name of data base and, where practicable, search terms used)

CNABS, CNTXT, DWPI, VEN, USTXT, EPTXT, WOTXT, CNKI, GOOGLE, STN, 汉防己甲素, 汉防己碱, 粉防己碱, 粉防己甲素, 汉甲素, 千金藤碱, 苄基异喹啉, 冠状病毒, 新冠, 高血压, tetrandrine, hanfangchin, fanchinine, cepharanthine, benzylisoquinoline, COVID-19, 2019-ncov, SARS, coronavirus, neo-coronary pneumonia, 518-34-3, 5990-67-0, 6490-80-8, 916770-74-6, 607379-81-7, 1263-79-2, 477-57-6, 5956-77-4, 1421757-32-5

**C. DOCUMENTS CONSIDERED TO BE RELEVANT**

| Category* | Citation of document, with indication, where appropriate, of the relevant passages | Relevant to claim No. |
|---|---|---|
| X | KIM Dong Eon et al. "Natural Bis-Benzylisoquinoline Alkaloids-Tetrandrine, Fangchinoline, and Cepharanthine, Inhibit Human Coronavirus OC43 Infection of MRC-5 Human Lung Cells" *Biomolecules,* Vol. 9, 04 November 2019 (2019-11-04), the abstract | 1-3, 7-22, 30-33 |
| X | SHEN, Liang et al. "High-Throughput Screening and Identification of Potent Broad-Spectrum Inhibitors of Coronaviruses" *Journal of Virology,* Vol. 93, No. 12, 29 May 2019 (2019-05-29), table 1 | 1-3, 7-22, 30-33 |
| X | QU, Xiuyuan et al. "Characterization of Spike Glycoprotein of 2019-nCoV on Virus Entry and Its Immune Cross-reactivity with Spike Glycoprotein of SARS-CoV" *https://www.researchgate.net/publication/340192069_Characterization_of_spike_glycoprotein_of_2019-nCoV_on_virus_entry_and_its_immune_cross-reactivity_with_spike_glycoprotein_of_SARS-CoV,* 28 February 2020 (2020-02-28), p. 8, lines 7-9 | 1-33 |

☑ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 November 2020** | **07 December 2020** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/101581**

**C. DOCUMENTS CONSIDERED TO BE RELEVANT**

| Category* | Citation of document, with indication, where appropriate, of the relevant passages | Relevant to claim No. |
|---|---|---|
| X | RUAN, Zijing et al. "Potential Inhibitors Targeting RNA-dependent RNA Polymerase Activity (NSP12) of SARS-CoV-2" *https://europepmc.org/article/ppr/ppr115608,* 02 March 2020 (2020-03-02), p. 11, the eighth line from the bottom to p. 12, line 4 | 1-33 |
| PX | JEON Sangeun et al. "Identification of Antiviral Drug Candidates against SARS-CoV-2 from FDA-Approved Drugs" *Antimicrobial, Agents and Chemotherapy,* Vol. 64, No. 7, 23 June 2020 (2020-06-23), p. 1, the fourth line from the bottom to p. 2, line 5 | 1-33 |
| PX | LECHOWICZ Kacper et al. "COVID-19: The Potential Treatment of Pulmonary Fibrosis Associated with SARS-CoV-2 Infection" *Journal of Clinical Medicine,* Vol. 9, 19 June 2020 (2020-06-19), table 3 | 1-33 |
| PX | HENAN PROVINCIAL PEOPLE'S HOSPITAL. "Tetrandrine Tablets Used in the Treatment of COVID-19 (TT-NPC)" *https://clinicaltrials.gov/ct2/show/NCT04308317,* 16 March 2020 (2020-03-16), study description section | 1-33 |
| A | 李丹等 (LI, Dan et al.). "苄基异喹啉类生物碱的药理活性研究进展 (Advances in Pharmacological Activities of Benzylisoquinoline Alkaloids)" *广东化工 (Guangdong Chemical Industry),* Vol. 44, No. 9, 31 December 2017 (2017-12-31), the abstract | 1-33 |

**INTERNATIONAL SEARCH REPORT**

International application No.
**PCT/CN2020/101581**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**

International application No.
**PCT/CN2020/101581**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: 权利要求**20-31**，**33**（部分）
because they relate to subject matter not required to be searched by this Authority, namely:

[1] Claim 20 relates to a method for the prevention and/or treatment of disease or infection caused by coronavirus or disease or infection caused by coronavirus and accompanied by hypertension, claim 21 relates to a method for the prevention and/or treatment of pulmonary disease or symptoms associated with coronavirus or pulmonary disease or symptoms associated with coronavirus and accompanied by hypertension, claim 22 relates to a method for the inhibition of coronavirus replication or propagation in a host in need, claims 23-31 and 33 refer directly or indirectly to claims 20-22, and the above methods are methods of treatment of disease and do not comply with PCT Implementing Rule 39.1(iv). This search report is made on the basis of the subject name of claims 20-31 and 33 modified as follows: "the use of prophylactic and/or effective amounts of the compound shown in general formula I, its geometrical isomers or pharmaceutically acceptable salts and/or solvates and/or hydrates thereof, or comprising the compound shown in general formula I, the pharmaceutical compositions of its geometrical isomers or pharmaceutically acceptable salts and/or solvates and/or hydrates thereof, in the preparation of products for the prevention and/or treatment of diseases or infections caused by coronaviruses or diseases or infections caused by coronaviruses and accompanied by hypertension; the use of the prophylactic and/or effective amounts of a compound shown in general formula I, its geometrical isomers or pharmaceutically acceptable salts and/or solvates and/or hydrates thereof, or comprising a compound shown in general formula I, the pharmaceutical compositions of its geometrical isomers or pharmaceutically acceptable salts and/or solvates and/or hydrates thereof in the preparation of products for the prevention and/or treatment of pulmonary diseases or symptoms associated with coronaviruses or pulmonary diseases or symptoms associated with coronaviruses and accompanied by hypertension; the use of the prophylactic and/or effective amounts of a compound shown in general formula I, its geometrical isomers or pharmaceutically acceptable salts and/or solvates and/or hydrates thereof, or comprising the compound shown in general formula I, the pharmaceutical compositions of its geometrical isomers or pharmaceutically acceptable salts and/or solvates and/or hydrates thereof in the preparation of products for the inhibition of coronavirus replication or propagation".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202010140356

### Non-patent literature cited in the description


- **ZAKI AM ; VAN BOHEEMEN S ; BESTEBROER T M et al.** Isolation of a novel coronavirus from a man with pneumonia in Saudi Arabia[J. *N Engl J Med,* 2012, vol. 367 (19), 1814-1820
- **ZHONG NS ; ZHENG BJ ; LI YM et al.** Epidemiology and cause of severe acute respiratory syndrome (SARS) in Guangdong, People's Republic of China, in February, 2003[J. *The Lancet,* 2003, vol. 362 (9393), 1353-1358
- **CUI J ; LI F ; SHI ZL.** Origin and evolution of pathogenic coronaviruses[J]. *Nat Rev Microbiol,* 2019, vol. 17 (3), 181-192
- **WANG M ; CAO R ; ZHANG L et al.** Remdesivir and chloroquine effectively inhibit the recently emerged novel coronavirus (2019-nCoV) in vitro[J. *Cell Res,* 2020, vol. 0, 1-3